# FASCICULE DE BREVET EUROPEEN

(11) **EP 1 419 762 B1**
(45) Date de publication et mention de la délivrance du brevet: **06.06.2007**
(21) Numéro de dépôt: 03292574.5
(22) Date de dépôt: 16.10.2003
(51) Int. Cl.: A61K 8/04, A61K 8/06, A61K 8/25, A61K 8/26, A61K 8/81, A61Q 1/02, A61Q 19/00

(54) **Procédé de traitement des peaux grasses basé sur l'application d'une composition comprenant une charge composite silice-alumine et un hydrocolloide épaississant**
Behandlung von fettiger Haut mit einer Zusammensetzung enthaltend einen Aluminiumoxyd-Silizium Verbundwerkstoff und ein verdickendes Hydrokolloid
Treatment of greasy skin based on the application of a composition comprising a silica-alumina composite and a thickening hydrocolloid

(30) Priorité: 12.11.2002 FR 0214117
(43) Date de publication de la demande: 19.05.2004
(73) Titulaire: L'ORÉAL, 75008 Paris (FR)
(72) Inventeur: Cassin, Guillaume, 91140 Villebon sur Yvette (FR)
(74) Mandataire: Kromer, Christophe

(56) Documents cités:
- EP-A- 0 682 939
- EP-A- 1 066 818
- FR-A- 2 167 931

## Description

La présente invention se rapporte à un procédé de traitement cosmétique des peaux grasses, comprenant l'application topique sur la peau d'une composition renfermant, dans un milieu physiologiquement acceptable, une dispersion de particules colloïdales d'au moins une charge composite silice-alumine et au moins un hydrocolloïde épaississant donné.

La brillance de la peau, souvent liée à une sécrétion importante de sébum, est un problème affectant plus particulièrement les adolescents mais qui peut aussi se manifester à l'âge adulte sous l'effet notamment d'une hyperproduction d'androgènes. Elle peut aussi être liée à la sueur résultant d'une activité physique ou des conditions climatiques. Or, une peau brillante est considérée comme inesthétique, d'autant plus qu'elle entraîne souvent une moins bonne tenue du maquillage qui a tendance à se dégrader visuellement au cours de la journée.

Les compositions classiques dites matifiantes contiennent généralement des poudres absorbant le sébum et l'huile excédentaire de la composition non absorbée par la peau. Parmi les poudres matifiantes d'origine naturelle ou synthétique, on peut citer notamment les charges telles que le talc, l'amidon, le mica, la silice, les poudres de nylon, les poudres de polyéthylène, la poly-bêta-alanine, les poudres de poly(méth)acrylate de méthyle. Ce type de charges présente l'inconvénient de donner à la peau un aspect poudreux, pas naturel, qui peut même accentuer les défauts de la peau. De plus, les compositions les contenant sont généralement desséchantes à long terme et s'étalent difficilement. Leur effet matifiant est peu durable dans le temps. Enfin, plusieurs de ces charges ont tendance à sédimenter, sauf à être formulées dans des compositions relativement visqueuses.

En outre, il a été proposé par la Demanderesse d'utiliser comme agents matifiants des copolymères vinylpyrrolidone / 1-triacontène (FR-2 820 972) ou des particules de résine de mélamine-formaldéhyde ou d'urée-formaldéhyde (FR-2 792 642), de styrène acrylique (FR-2 801 215) ou de polytétrafluoroéthylène (FR-2 820 977). Ces agents matifiants confèrent une certaine opacité aux compositions les contenant, alors que les compositions transparentes sont de plus en plus recherchées par les consommateurs.

La Demanderesse a également divulgué dans la demande EP-0 682 939 des agents matifiants constitués de dispersions colloïdales de particules inorganiques, en particulier de silice, qui peuvent être préparées par un procédé sol-gel. Ces agents matifiants permettent, certes, de réaliser des compositions transparentes mais ils présentent l'inconvénient de déstabiliser les compositions les contenant, en présence d'un hydro-colloïde épaississant, en particulier à pH acide.

Or, il est souvent utile de prévoir des épaississants dans les compositions cosmétiques afin d'obtenir des textures faciles à appliquer et agréables au toucher. En particulier, l'utilisation d'épaississants hydrosolubles ou hydrodispersibles est particulièrement utile dans des produits pour peaux grasses qui sont formulés à base d'eau pour ne pas accentuer la brillance de la peau et à un pH acide proche de celui de la peau pour assurer une tolérance optimale.

La Demanderesse a maintenant découvert que l'utilisation d'une dispersion aqueuse d'une charge composite silice-alumine permettait de formuler des produits pour peaux grasses qui aient un bon pouvoir matifiant tout en offrant une bonne compatibilité physiologique, en terme de pH, et une bonne cosméticité en ce sens que ces produits peuvent se trouver sous forme de compositions transparentes gélifiées conservant leur aspect pendant plusieurs années.

Ces charges composites ont notamment été décrites dans le brevet US-5,118,727 en tant qu'agent liant pour la fabrication de moules en céramique destinés à la production de pièces métalliques, et dans le brevet US-2,892,797, dans des compositions telles que des émulsions de peinture, des catalyseurs, des caoutchoucs ou encore des compositions pour le traitement des textiles. Elles ont également été décrites par la Demanderesse pour lisser les rides et ridules par effet tenseur (demande non publiée).

Elles ont également été décrites dans la demande WO 90/01919 comme matière première pour l'encapsulation de polymères destinés à la coloration artificielle de la peau. Dans cette application, la solution de sol de silice composite utilisée est gélifiée par ajout de chlorure de calcium, de sorte qu'elle ne se trouve plus sous forme de dispersion colloïdale.

Ces documents ne suggèrent pas d'utiliser une dispersion colloïdale de particules composites silice-alumine pour la préparation de compositions destinées au soin des peaux grasses.

De son côté, la demande FR-2 167 931 décrit une composition pour le soin de la peau, en particulier à effet anti-rides, comprenant une protéine dérivée de kératines et une dispersion colloïdale de silice qui peut être un composite silice-alumine. Lorsqu'elle est utilisée sur une peau grasse, cette composition est rincée après application, pour éliminer le sébum émulsionné et le dépôt blanchâtre formé par la silice sur la peau, puis une seconde couche est appliquée.

Toutefois, la composition décrite dans ce document ne renferme pas d'hydro-colloïde épaississant au sens de la présente invention. Il n'est donc pas prévisible à la lecture de ce document que les dispersions colloïdales de composite silice-alumine puissent permettre de formuler des compositions stables en présence de ces hydrocolloïdes.

La présente invention a donc pour objet un procédé de traitement cosmétique des peaux grasses, comprenant l'application topique sur la peau d'une composition renfermant, dans un milieu physiologiquement acceptable, une dispersion de particules colloïdales d'au moins une charge composite silice-alumine et au moins un hydrocolloïde épaississant choisi parmi : les polymères carboxyvinyliques modifiés ou non ; les polyacrylates et polyméthacrylates ; les polyacrylamides ; les polymères et copolymères d'acide 2-acrylamido 2-méthylpropane sulfonique, éventuellement réticulés et/ou neutralisés et/ou rendus hydrophobes par greffage ; les copolymères anioniques réticulés d'acrylamide et d'acide 2-acrylamido 2-méthylpropane sulfonique ; et les biopolymères polysaccharidiques.

Par "particules colloïdales" au sens de la présente invention, on entend une dispersion de particules ayant un diamètre moyen en nombre compris entre 3 et 150 nm, de préférence entre 5 et 30 nm, mieux, entre 10 et 15 nm. Ces particules conservent les diamètres précités dans la composition les contenant, sans s'aggréger, et n'ont donc pas de propriétés épaississantes en ce sens qu'une composition constituée de 15% en poids de particules colloïdales selon l'invention dans l'eau présente une viscosité inférieure à 0,05 Pa.s pour un taux de cisaillement égal à 10⁻¹s, la viscosité étant mesurée à 25°C à l'aide d'un rhéomètre RheoStress RS150 de HAAKE en configuration cône-plan, le cône ayant un diamètre de 60 mm et un angle de 2°. Par ailleurs, les dispersions de particules selon l'invention sont transparentes, en ce sens que la turbidité de compositions constituées de particules à 1% en poids dans l'eau est inférieure à 200 NTU, la mesure étant réalisée à 25°C à l'aide d'un turbidimètre Hach 2100P. Cette propriété des particules à former une solution colloïdale est liée à leur potentiel zêta qui est de préférence inférieur à -20 mV, et plus préférentiellement inférieur à -25 mV, à pH 7 et à 25°C, tel que mesuré à l'aide d'un appareil DELSA 440SX de COULTER Scientific Instrument.

Par "particules de charge composite silice-alumine" au sens de la présente invention, on entend des particules constituées d'oxyde de silicium et dont la surface a été modifiée chimiquement de façon à remplacer certains au moins des atomes de silicium par des atomes d'aluminium formant au plus une couche monomoléculaire d'aluminium. La portion de surface de ces particules qui est recouverte d'aluminium est généralement comprise entre 1 et 100%, de préférence entre 1 et 10%, mieux, entre 4 et 6%.

Ces particules peuvent être préparées, notamment, comme décrit dans le brevet US-2,892,797, en mélangeant un sol de silice avec un aluminate de sodium. Elles sont par ailleurs disponibles dans le commerce auprès de la société GRACE sous la référence commerciale Ludox AM^{®} sous forme de dispersions aqueuses à 30% en poids de matière active.

La composition selon l'invention est adaptée à une application topique sur la peau et comprend donc généralement un milieu physiologiquement acceptable, c'est-à-dire compatible avec la peau et/ou ses phanères. Ainsi, la composition selon l'invention a de préférence un pH inférieur à 7, mieux, inférieur à 6.

La quantité de particules de charge composite présentes dans la composition peut varier dans une large mesure en fonction de l'effet recherché. A titre d'exemple, ces particules peuvent représenter (en matière active) de 0,01 à 10% en poids, et de préférence de 0,1 à 5% en poids, par rapport au poids total de la composition.

Comme indiqué précédemment, outre la dispersion colloïdale de composite silice-alumine, la composition selon l'invention renferme au moins un hydrocolloïde épaississant.

Par "hydro-colloïdes épaississants", on entend des polymères hydrosolubles ou hydrodispersibles comprenant au moins 20 unités monomériques et qui, à une concentration de 0,2% en poids dans un milieu aqueux, alcoolique ou hydro-alcoolique confèrent à ce milieu une viscosité supérieure à 5 mPa.s pour un taux de cisaillement égal à 10s⁻¹, la mesure étant réalisée à 25°C à l'aide d'un rhéomètre RheoStress RS2150 de la société HAAKE en configuration cône-plan, le cône de mesure ayant un diamètre de 60 mm et un angle de 2°. Les polymères en question ont généralement un poids moléculaire en nombre moyen allant de 1.000 à 20.000.000 g/mol, de préférence de 20.000 à 10.000.000 g/mol et plus préférentiellement encore de 100.000 à 800.000 g/mol.

Des exemples de tels hydro-colloïdes utilisables dans la composition selon l'invention comprennent : les polymères carboxyvinyliques modifiés ou non, tels que les produits commercialisés sous les dénominations Carbopol (nom CTFA : carbomer) et Pemulen (nom CTFA : Acrylates/C₁₀-₃₀ akyl acrylate crosspolymer) par la société Goodrich ; les polyacrylates et polyméthacrylates tels que les produits vendus sous les dénominations de Lubrajel et Norgel par la société GUARDIAN ou sous la dénomination Hispagel par la société HISPANO CHIMICA ; les polyacrylamides ; les polymères et copolymères d'acide 2-acrylamido 2-méthylpropane sulfonique, éventuellement réticulés et/ou neutralisés et/ou rendus hydrophobes par greffage, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ; les copolymères anioniques réticulés d'acrylamide et de poly(acide 2-acrylamido 2-méthylpropane sulfonique) (AMPS), se présentant par exemple sous la forme d'une émulsion E/H, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les biopolymères polysaccharidiques comme la gomme de xanthane, la gomme de guar, la gomme de caroube, la gomme d'acacia, les scléroglucanes, les dérivés de chitine et de chitosane, les carraghénanes, les gellanes, les alginates, les celluloses telles que la cellulose microcristalline, la carboxyméthylcellulose, l'hydroxyméthylcellullose et l'hydroxypropylcellulose ; et leurs mélanges.

La composition selon l'invention peut par exemple contenir de 0,1 à 10% et, mieux, de 0,2 à 6% de tels hydro-colloïdes, par rapport au poids total de la composition.

La composition selon l'invention peut se présenter sous toutes les formes galéniques classiquement utilisées pour une application topique et notamment sous forme de gels aqueux. Elles peut aussi, par ajout d'une phase grasse ou huileuse, se présenter sous forme de dispersions ou d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition se présente sous forme d'une émulsion huile-dans-eau ou d'un gel aqueux.

Dans le cas où elle est formulée sous la forme d'une émulsion, la proportion de la phase huileuse de l'émulsion peut aller par exemple de 0,5 à 20 % en poids, par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition.

Dans le cas où elle se trouve sous la forme d'une émulsion H/E, la composition selon l'invention peut contenir, comme tensioactifs, au moins un composé choisi parmi : les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénées et/ou oxypropylénées, tels que les esters de glycéryle et d'acide gras en C₈-C₂₄, et leurs dérivés oxyalkylénés, les esters de sorbitol et d'acide gras en C₈-C₂₄, et leurs dérivés oxyalkylénés, les esters de sucre (sucrose, glucose, alkylglucose) et d'acide gras en C₈-C₂₄, et leurs dérivés oxyalkylénés ; les esters de polyéthylène glycol et d'acide gras en C₈-C₂₄, et leurs dérivés oxyalkylénés ; les éthers de polyalkylène glycol et d'alcools gras en C₈-C₂₄; les éthers de sucre et d'alcools gras en C₈-C₂₄, et leurs mélanges.

En variante, la composition selon invention sous forme d'émulsion peut renfermer un polymère amphiphile ionique et être exempte d'émulsionnant.

La composition selon l'invention peut également contenir les adjuvants habituels dans les domaines cosmétique et dermatologique, tels que les gélifiants hydrophiles ou lipophiles, les actifs, les conservateurs, les solvants, les parfums, les charges, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse ou dans la phase aqueuse. Ces adjuvants, ainsi que leurs concentrations, doivent être tels qu'ils ne nuisent pas aux propriétés avantageuses de la charge composite selon l'invention.

Comme actifs, la composition selon l'invention contiendra de préférence au moins un actif choisi parmi : les rétinoïdes et en particulier le rétinol ; les sels de zinc tels que le gluconate de zinc ; un extrait de Laminaria saccharina ; un extrait d'igname sauvage ; le triclosan ; le phénoxyéthanol ; l'octoxyglycérine ; l'octanoylglycine ; un extrait de clou de girofle ; le caprylyl glycol ; l'acide azélaïque ; les α-hydroxyacides tels que les acides lactique ou glycolique : les β-hydroxyacides, en particulier l'acide salicylique et ses dérivés tels que l'acide n-octanoyl-5-salicylique ; l'acide ursolique ; le panthénol ; le niacinamide et l'octopirox.

Comme charges, on peut citer par exemple, les particules de polyamide (Nylon) et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m³), 551 DE 20 (granulométrie d'environ 30 µm et masse volumique 65 kg/m³), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les fibres de polyamide ; les microbilles de résine de silicone telles que celles commercialisées sous la dénomination TOSPEARL par la société Toshiba Silicone, notamment TOSPEARL 240 ; la silice ; les oxydes métalliques tels que le dioxyde de titane, l'oxyde de zinc, l'alumine ; le mica ; le talc ; la séricite ; le nitrure de bore ; les argiles ; et leurs mélanges.

L'invention sera maintenant illustrée par les exemples non limitatifs suivants. Dans ces exemples, les quantités sont indiquées en pourcentage pondéral.

### EXEMPLES

### Exemple comparatif 1 : composition cosmétique

| | |
|---|---|
| Eau | 80.42 g |
| Glycérine | 1.00 g |
| Phénoxyéthanol | 1.00 g |
| Acide lactique | 0.06 g |
| Gellane | 0.20 g |
| Ethanol | 10.00g |
| Dispersion de particules colloïdales de silice (Cosmo S40 de Catalyst and Chemicals)* | 7.32g |

| | |
|---|---|
| * dispersion aqueuse à 41% de matière active de silice ayant une taille de particule de 18 nm | |

### Exemple comparatif 2 : composition cosmétique

| | |
|---|---|
| Eau | 80.02 g |
| Glycérine | 1.00 g |
| Phenoxyéthanol | 1.00 g |
| Acide lactique | 0.06 g |
| Ethanol | 10.00 g |
| Dispersion de particules colloïdales de silice (Cosmo S40 de Catalyst and Chemicals)* | 7.32 g |
| Polymère carboxyvinylique (Carbopol 980) | 0.30 g |
| Triéthanolamine | 0.30g |

| | |
|---|---|
| * dispersion aqueuse à 41% de matière active de silice ayant une taille de particule de 18 nm | |

### Exemple 1 : composition cosmétique

| | |
|---|---|
| Eau | 77.74 g |
| Glycérine | 1.00 g |
| Phenoxyéthanol | 1.00 g |
| Acide lactique | 0.06 g |
| Ethanol | 10.00 g |
| Gellane | 0.20 g |
| Dispersion de particules colloïdales composites de | |
| silice alumine (Ludox AM de GRACE) | 10.00 g |

### Exemple 2: composition cosmétique

| | |
|---|---|
| Eau | 77.34 g |
| Glycérine | 1.00 g |
| Phenoxyéthanol | 1.00 g |
| Acide lactique | 0.06 g |
| Ethanol | 10.00 g |
| Dispersion de particules colloïdales composites de silice alumine (Ludox AM de GRACE) | 10.00 g |
| Polymère carboxyvinylique (Carbopol 980) | 0.30 g |
| Triéthanolamine | 0.30 g |

### Exemple 3 : composition cosmétique

| | |
|---|---|
| Eau | 63.82 g |
| Stéarate de polyglycéryle | 1.00 g |
| Mono stéarate de sorbitane oxyéthyléné | 0.75 g |
| Sel disodique de stéaroyl glutamate | 0.50 g |
| Stéarate d'isocétyle | 5.00 g |
| Triglycérides caprylique/caprique | 2.00 g |
| Isononanoate d'isononyle | 3.00 g |
| Butylparaben | 0.25 g |
| Chlorophénésine | 0.25 g |
| Acide lactique | 0.10 g |
| Dispersion de particules colloïdales composites de | |
| silice alumine (Ludox AM de GRACE) | 23.33 g |

### Exemple 4 : mise en évidence de l'effet matifiant des compositions selon l'invention

Les compositions des Exemples 1 et 3 ont été testées sur un panel de sept femmes ayant une peau grasse et présentant des irrégularités du micro-relief cutané. On a observé une diminution instantanée de la brillance de la peau après application de ces compositions.

### Exemple 5 : test de stabilité

On a comparé la stabilité après deux mois de conservation à 4°C, 25°C, 37°C et 45°C des compositions des Exemples comparatifs 1 et 2 et des Exemples 1 à 3 ci-dessus.

Une composition était considérée comme stable si l'ensemble des caractéristiques macroscopiques (couleur, odeur, répartition de ses phases, viscosité, pH) et microscopiques de la composition restaient inchangées. Elle était considérée comme instable si l'une au moins de ces caractéristiques était modifiée.

Les résultats sont rassemblés dans le Tableau 1 ci-dessous :

**Tableau 1**

| Test de stabilité | | |
|---|---|---|
| Composition | pH | Stabilité |
| Exemple comparatif 1 *(à 3% de silice)* | 5,7 | instable |
| Exemple comparatif 2 *(à 3% de silice)* | 6,7 | instable |
| Exemple 1 *(à 3% de silice composite)* | 5,1 | stable |
| Exemple 2 *(à 3% de silice composite)* | 6,3 | stable |
| Exemple 3 *(à 7% de silice composite)* | 6,8 | stable |

Il ressort du Tableau 1 ci-dessus que les compositions selon l'invention, qui comprennent des particules composites silice-alumine, sont stables à pH inférieur à 7, et même inférieur à 6, y compris en présence de polymères épaississants, alors que tel n'est pas le cas des compositions renfermant des particules colloïdales de silice ne se trouvant pas sous forme de composite, qui ont tendance à prendre en masse au bout d'un mois de stockage.

## Revendications

1. Procédé de traitement cosmétique des peaux grasses, comprenant l'application topique sur la peau d'une composition renfermant, dans un milieu physiologiquement acceptable, une dispersion de particules colloïdales d'au moins une charge composite silice-alumine et au moins un hydrocolloïde épaississant choisi parmi : les polymères carboxyvinyliques modifiés ou non les polyacrylates et polyméthacrylates ; les polyacrylamides ; les polymères et copolymères d'acide 2-acrylamido 2-méthylpropane sulfonique, éventuellement réticulés et/ou neutralisés et/ou rendus hydrophobes par greffage : les copolymères anioniques réticulés d'acrylamide et d'acide 2-acrylamido 2-méthylpropane sulfonique ; et les biopolymères polysaccharidiques.

2. Procédé selon la revendication 1, **caractérisé en ce que** lesdites particules ont un diamètre moyen en nombre compris entre 5 et 30 nm.

3. Procédé selon la revendication 2, **caractérisé en ce que** lesdites particules ont un diamètre moyen en nombre compris entre 10 et 15 nm.

4. Procédé selon l'une quelconque des revendications 1 à 3, **caractérisé en ce que** la surface desdites particules qui est recouverte d'aluminium est comprise entre 4 et 6%.

5. Procédé selon l'une quelconque des revendications 1 à 4, **caractérisé en ce que** lesdites particules ont un potentiel zêta inférieur à -25 mV, à pH 7 et à 25°C.

6. Procédé selon l'une quelconque des revendications 1 à 5, **caractérisé en ce que** la composition a un pH inférieur à 7.

7. Procédé selon la revendication 6, **caractérisé en ce que** la composition a un pH inférieur à 6.

8. Procédé selon l'une quelconque des revendications 1 à 7, **caractérisé en ce que** les biopolymères polysaccharidiques sont choisis parmi la gomme de xanthane, la gomme de guar, la gomme de caroube, la gomme d'acacia, les scléroglucanes, les dérivés de chitine et de chitosane, les carraghénanes, les gellanes, les alginates, les celluloses telles que la cellulose microcristalline, la carboxyméthylcellulose, l'hydroxyméthylcellullose et l'hydroxypropylcellulose ; et leurs mélanges.

9. Procédé selon la revendication 8, **caractérisé en ce que** la composition est sous forme d'émulsion huile-dans-eau ou de gel aqueux.

10. Procédé selon l'une quelconque des revendications 1 à 9, **caractérisé en ce que** la composition renferme de 0,1 à 10 % en poids de particules colloïdales de charge composite silice-alumine.

## Claims

1. Method for the cosmetic treatment of greasy skin, comprising the topical application to the skin of a composition including, in a physiologically acceptable medium, a dispersion of colloidal particles of at least one silica/alumina composite filler and at least one thickening hydrocolloid chosen from: modified or unmodified carboxyvinyl polymers; polyacrylates and polymethacrylates; polyacrylamides; 2-acrylamido-2-methylpropanesulphonic acid polymers and copolymers optionally crosslinked and/or neutralized and/or rendered hydrophobic by grafting; crosslinked anionic copolymers of acrylamide and of 2-acrylamido-2-methylpropanesulphonic acid; and polysaccharide biopolymers.

2. Method according to Claim 1, **characterized in that** the said particles have a number-average diameter of between 5 and 30 nm.

3. Method according to Claim 2, **characterized in that** the said particles have a number-average diameter of between 10 and 15 nm.

4. Method according to any one of Claims 1 to 3, **characterized in that** the surface area of the said particles which is covered with aluminium is between 4 and 6%.

5. Method according to any one of Claims 1 to 4, **characterized in that** the said particles have a zeta potential of less than -25 mV, at pH 7 and at 25°C.

6. Method according to any one of Claims 1 to 5, **characterized in that** the composition has a pH of less than 7.

7. Method according to Claim 6, **characterized in that** the composition has a pH of less than 6.

8. Method according to any one of Claims 1 to 7, **characterized in that** the polysaccharide biopolymers are chosen from xanthan gum, guar gum, locust bean gum, gum acacia, scleroglucans, chitin and chitosan derivatives, carrageenans, gellans, alginates, celluloses, such as microcrystalline cellulose, carboxymethylcellulose, hydroxymethylcellulose and hydroxypropylcellulose, and their mixtures.

9. Method according to Claim 8, **characterized in that** the composition is in the form of an oil-in-water emulsion or of an aqueous gel.

10. Method according to any one of Claims 1 to 9, **characterized in that** the composition includes from 0.1 to 10% by weight of colloidal particles of silica/alumina composite filler.

## Patentansprüche

1. Verfahren zur kosmetischen Behandlung von fettiger Haut, das die topische Anwendung einer Zusammensetzung, die in einem physiologisch akzeptablen Medium eine Dispersion von kolloidalen Partikeln mindestens eines Siliciumdioxid-Aluminiumoxid-Verbundmaterials und mindestens ein verdickendes Hydrokolloid enthält, das unter modifizierten oder nichtmodifizierten Carboxyvinylpolymeren; Polyacrylaten und Polymethacrylaten; Polyacrylamiden; Polymeren und Copolymeren von 2-Acrylamido-2-methylpropansulfonsäure, die gegebenenfalls vernetzt und/oder neutralisiert und/oder durch Pfropfen hydrophob gemacht sind; vernetzten anionischen Copolymeren von Acrylamid und 2-Acrylamido-2-methylpropansulfonsäure; und Polysaccharid-Biopolymeren ausgewählt ist; auf die Haut umfasst.

2. Verfahren nach Anspruch 1, **dadurch gekennzeichnet, dass** die Partikel einen zahlenmittleren Durchmesser von 5 bis 30 nm aufweisen.

3. Verfahren nach Anspruch 2, **dadurch gekennzeichnet, dass** die Partikel einen zahlenmittleren Durchmesser von 10 bis 15 nm aufweisen.

4. Verfahren nach einem der Ansprüche 1 bis 3, **dadurch gekennzeichnet, dass** die Oberfläche der Partikel, die mit Aluminium bedeckt ist, im Bereich von 4 bis 6 % liegt.

5. Verfahren nach einem der Ansprüche 1 bis 4, **dadurch gekennzeichnet, dass** die Partikel bei pH 7 und 25 °C ein Zeta-Potential unter -25 mV besitzen.

6. Verfahren nach einem der Ansprüche 1 bis 5, **dadurch gekennzeichnet, dass** die Zusammensetzung einen pH-Wert unter 7 aufweist.

7. Verfahren nach Anspruch 6, **dadurch gekennzeichnet, dass** die Zusammensetzung einen pH-Wert unter 6 hat.

8. Verfahren nach einem der Ansprüche 1 bis 7, **dadurch gekennzeichnet, dass** die Polysaccharid-Biopolymere unter Xanthan, Guargummi, Johannisbrotkernmehl, Akaziengummi, Skleroglucanen, Chitin- und Chitosanderivaten, Carrageenanen, Gellanen, Alginaten, Cellulosen, wie mikrokristalliner Cellulose, Carboxymethylcellulose, Hydroxymethylcellulose und Hydroxypropylcellulose; und deren Gemischen ausgewählt sind.

9. Verfahren nach Anspruch 8, **dadurch gekennzeichnet, dass** die Zusammensetzung in Form einer Öl-in-Wasser-Emulsion oder als wässriges Gel vorliegt.

10. Verfahren nach einem der Ansprüche 1 bis 9, **dadurch gekennzeichnet, dass** die Zusammensetzung 0,1 bis 10 Gew.-% der kolloidalen Partikel des Siliciumdioxid-Aluminiumoxid-Verbundmaterials enthält.
